# EUROPEAN PATENT APPLICATION

(11) **EP 4 354 144 A1**
(43) Date of publication of application: **17.04.2024**
(21) Application number: 22860631.5
(22) Date of filing: 26.08.2022
(51) Int. Cl.: G01N 33/68, G01N 33/58, G01N 33/543, B01L 3/00

(54) **MICROFLUIDIC CHIP FOR TESTING PROTEIN ANTIGEN, AND METHOD, KIT AND SYSTEM**

(30) Priority: 26.08.2021 CN 202110987339
(71) Applicant: Shenzhen Yhlo Biotech Co., Ltd., Shenzhen, Guangdong 518116 (CN)
(72) Inventor: CHENG, Bangning, Shenzhen, Guangdong 518116 (CN); LI, Shunji, Shenzhen, Guangdong 518116 (CN); LIU, Bifeng, Shenzhen, Guangdong 518116 (CN); QIAN, Chungen, Shenzhen, Guangdong 518116 (CN); XIAO, Yujin, Shenzhen, Guangdong 518116 (CN); CHEN, Peng, Shenzhen, Guangdong 518116 (CN)
(74) Representative: Mewburn Ellis LLP
(86) International application number: PCT/CN2022/115133
(87) International publication number: WO 2023/025285

(57) **Abstract**

The present invention relates to a microfluidic chip, a method, a reagent kit, and a system for detecting protein antigens. The method for detecting protein antigens includes the following steps: mixing a test sample with a reaction reagent, and forming the mixture of the test sample and the reaction reagent into a plurality of liquid droplets through microfluidic control technology, wherein the reaction reagent includes marker-labeled antibodies and an electrolyte providing agent, the marker-labeled antibodies are adapted to specifically bind to protein antigens in the test sample, forming the plurality of antigen-antibody complexes, the plurality of antigen-antibody complexes are encapsulated within the liquid droplets, and the plurality of antigen-antibody complexes are capable of undergoing agglutination reaction under an action of the electrolyte providing agent; and after the agglutination reaction, detecting and analyzing signal intensities of the liquid droplets corresponding to the agglutination reaction, thereby determining the quantity of the protein antigens in the test sample. The detection method is simple, not requiring washing steps and the plurality of rounds of sample additions, consumes a decreased amount of reagent with high reproducibility.

## Description

### TECHNICAL FIELD

The present invention relates to the field of immunodetection technology, particularly to a microfluidic chip, a method, a reagent kit, and a system for detecting protein antigens.

### BACKGROUND

The digitized microfluidic analysis technology based on microdroplets is an analytical technology rapidly developed in recent years. Its primary application is in digital polymerase chain reaction (dPCR). The foremost advantage of the droplet microfluidic control technology is the capability of generating droplets in milliseconds, producing thousands of monodispersed droplets within several seconds while precisely controlling the droplet size in the level of nanoliters or even picoliters. The droplet microfluidic control enables independent manipulation of individual droplets for mixing, transportation, and analysis, and can rapidly generate a great number of uniform droplets for a single experiment. This enables numerous parallel reactions to be conducted in a short time with high reproducibility. In microscopic view, the droplet has a large specific surface area, allowing rapid transfer of heat and substance within the droplet, facilitating expedition of the reactions. This technology in combination with the nucleic acid amplification of PCR and signal amplification can effectively complete a digital nucleic acid analysis.

Microfluidic immunological analysis integrates microfluidic control technology with an immunological analysis method by constructing an immunological analysis platform on a microfluidic chip, which can exert the advantages of the two analytical approaches. The reagent amount required in microfluidic analysis is extremely small, which significantly reduces the consumption of expensive immunological reagents such as antibodies. Fluid manipulation and integration at the micro- or nano-scale enhance the speed of an antigen-antibody reaction, effectively reducing reaction time. The distinctive biological recognition mechanism possessed by the immunological analysis method improves the specificity of the microfluidic analysis.

Consequently, in recent years, researchers have endeavored to apply droplet microfluidic chips to immunological analysis. However, unlike the detection of DNA or RNA, the detection of protein molecules requires biological signal markers for binding and reaction to determine the presence or absence of the substance, and the detection of antigens or antibodies primarily relies on a sandwich assay, which involves multiple rounds of sample additions and washing steps, resulting in complicated operations.

### SUMMARY

In view of this, there is a need to provide a simplified method for detecting protein antigens.

A method for detecting protein antigens includes the following steps:
mixing a test sample with a reaction reagent, and forming a mixture of the test sample and the reaction reagent into a plurality of liquid droplets through microfluidic control technology, wherein the reaction reagent includes marker-labeled antibodies and an electrolyte providing agent, the marker-labeled antibodies are adapted to specifically bind to protein antigens in the test sample, forming a plurality of antigen-antibody complexes, the plurality of antigen-antibody complexes are encapsulated within the liquid droplets, and the plurality of antigen-antibody complexes are capable of undergoing an agglutination reaction under an action of the electrolyte providing agent; and
after the agglutination reaction, detecting and analyzing signal intensities of the liquid droplets corresponding to the agglutination reaction, thereby determining the quantity of the protein antigens in the test sample.

In the above-described method for detecting protein antigens, through microfluidic control technology, a plurality of liquid droplets including antigen-antibody complexes and electrolyte are formed. Within the microenvironments of the liquid droplets, the plurality of antigen-antibody complexes undergo agglutination reaction due to the ions dissociated from the electrolyte. The quantity of the protein antigens in the test sample is determined by detecting the signal intensities corresponding to the agglutination reaction. Compared to a traditional protein antigen detection method that involves microfluidic control technology and a sandwich assay, the above-described detection method is simple, not requiring washing steps and multiple rounds of sample additions. Additionally, the detection method creates a microenvironment within each liquid droplet, and the liquid droplet has a large specific surface area in microscopic view. The heat and substance transfer in the liquid droplet requires less time, which is conducive to reduce reaction time, thereby expediting the immune reaction. In addition, the immune reaction time is short, and a minute change in reaction can be amplified and easily detected, thereby achieving a high sensitivity.

In an embodiment, the marker is latex microspheres, and the step of detecting signal intensities of the liquid droplets corresponding to the agglutination reaction after the agglutination reaction includes detecting turbidities of the liquid droplets.

In an embodiment, the marker is an aggregation-induced emission material, and the step of detecting signal intensities of the liquid droplets corresponding to the agglutination reaction after the agglutination reaction includes detecting luminous intensities of the liquid droplets.

In an embodiment, after the step of forming the mixture of the sample and the reaction reagent into the plurality of liquid droplets, the method further includes a step of detecting sizes of the liquid droplets formed from the test sample and the reaction reagent and screening out qualified liquid droplets according to the sizes of the liquid droplets.

In an embodiment, the reaction reagent further includes a fluorescent dye distinguishable from the marker; the step of detecting sizes of the liquid droplets formed from the test sample and the reaction reagent and screening out qualified liquid droplets according to the sizes of the liquid droplets includes:
detecting fluorescent distribution of the liquid droplets formed from the test sample and the reaction reagent; and
screening out the qualified liquid droplets based on the fluorescent distribution.

In an embodiment, the detection method is a quantitative detection method of the protein antigens, and the method further includes following steps:
mixing a standard sample for the protein antigens, having a same volume as the test sample, with the reaction reagent, and forming the mixture of the standard sample and the reaction reagent into a plurality of liquid droplets through microfluidic control technology;
after the agglutination reaction, detecting and totalizing signal intensities of the liquid droplets formed from the standard sample and the reaction reagent corresponding to the agglutination reaction, thereby obtaining a signal intensity of the standard sample;
after the agglutination reaction, detecting the signal intensities of the liquid droplets formed from the test sample and the reaction reagent corresponding to the agglutination reaction, totalizing the signal intensities of the liquid droplets formed from the test sample and the reaction reagent, thereby obtaining a signal intensity of the test sample; and
calculating a concentration of the protein antigens in the test sample from a concentration of the protein antigens in the standard sample, the signal intensity of the standard sample, and the signal intensity of the test sample.

A microfluidic chip for detecting protein antigens includes a droplet-forming component and a droplet channel. The droplet-forming component is configured to form a mixture of a sample and a reaction reagent into a plurality of liquid droplets. The droplet-forming component includes a first-phase channel and a reagent channel. The first-phase channel includes a first-phase inlet and a first-phase outlet. The first-phase inlet is in communication with the first-phase outlet. The reagent channel includes a reagent inlet and a reagent outlet in communication with the reagent inlet. The reagent outlet is located adjacent to the first-phase outlet, allowing a solution flowing out from the reagent outlet to be enveloped by a first phase flowing out from the first-phase outlet, thereby forming a liquid droplet.

In an embodiment, the microfluidic chip further includes a screening channel, a detection chamber, and a waste liquid reservoir. The screening channel is a three-way channel. One end of the screening channel is in communication with the droplet channel, and the other two ends of the screening channel are in communication with the detection chamber and the waste liquid reservoir respectively.

A protein antigen detection kit includes the above-described microfluidic chip for detecting protein antigens and a reaction reagent. The reaction reagent includes marker-labeled antibodies and an electrolyte providing agent. The marker-labeled antibodies are adapted to specifically bind to protein antigens in the test sample, forming antigen-antibody complexes. The electrolyte providing agent is configured to provide conditions for agglutination reaction of the antigen-antibody complexes.

A protein antigen detection system includes the above-described microfluidic chip and a detection component. The detection component is configured to detect signal intensities of the liquid droplets after the agglutination reaction within the microfluidic chip corresponding to the agglutination reaction.

### BRIEF DESCRIPTION OF THE DRAWINGS

FIG. 1 is a schematic view of an embodiment of a microfluidic chip for detecting protein antigens.
FIG. 2 is a schematic view of a reagent channel of the microfluidic chip shown in FIG. 1.
FIG. 3 is an enlarged view of portion A of the microfluidic chip shown in FIG. 1.
FIG. 4 is a schematic view of a detection chamber of the microfluidic chip shown in FIG. 1.
FIG. 5 is an enlarged view of portion B of the microfluidic chip shown in FIG. 1.

### Reference signs:

10. microfluidic chip; 110. first-phase channel; 120. reagent channel; 111. first-phase inlet; 113. first-phase outlet; 121. reagent outlet; 122. labeled antibody branch; 123. test sample branch; 124. auxiliary agent branch; 125. mixing branch; 130. droplet channel; 131. detection chamber; 132. detection hole; 140. second detection element; 141. screening element; 143. screening channel; 150. waste liquid reservoir.

### DETAILED DESCRIPTION

The present invention will be comprehensively described in order to facilitate the understanding of the present invention. The present invention can be implemented in many different forms and therefore is not limited to the embodiments described herein. On the contrary, the purpose of providing these embodiments is to make the understanding of the present invention more thorough and comprehensive.

It is to be noted that, an element, when referred to as being "fixed" or "connected" to another element, may be directly fixed or connected to the other element or via one or more intermediate elements. The terms such as "vertical", "horizontal", "left", "right", "up", "down", "inside", "outside", "bottom" and the like, indicating orientation or positional relationship, are generally based on orientation or positional relationship shown in the accompanying drawings, and used only for the purpose of facilitating the description, rather than indicating or implying that the related devices or elements must have the specific direction or be constructed and operated in the specific direction, and therefore not intended to limit the scope of the present application. In addition, the terms "first" and "second" are merely used for descriptive purposes, and should not be construed as indicating or implying a relative importance.

Unless otherwise specified, all the technical and scientific terms herein shall be understood as the same meaning with those commonly accepted by a person skilled in the art of the present invention. Such terms, as used herein, are for the purpose of describing exemplary examples of the present invention, not intended to limit the present application.

An embodiment of the present application provides a method for detecting protein antigens based on microfluidic droplet technology. The method for detecting protein antigens includes steps S100 and S200.

In step S 100, after a test sample and a reaction reagent are mixed together, the mixture of the test sample and the reaction reagent is formed into a plurality of liquid droplets through microfluidic control technology.

Specifically, the reaction reagent includes marker-labeled antibodies and an electrolyte providing agent. The marker-labeled antibodies are adapted to specifically bind to protein antigens in the test sample, thus forming a plurality of antigen-antibody complexes. The electrolyte providing agent, when dissolved in water, can dissociate into free ions to facilitate an agglutination reaction. In the microenvironment within the liquid droplet which contains the plurality of antigen-antibody complexes and a certain concentration of the electrolyte, the plurality of antigen-antibody complexes undergo agglutination reaction due to the ions dissociated from the electrolyte providing agent. As a result, the physical characteristics of the liquid droplet are changed, for instance, the turbidity is increased or the luminous intensity is enhanced. Therefore, after the agglutination reaction, by detecting the signal intensities of the liquid droplets corresponding to the agglutination reaction, it is possible to determine whether there are protein antigens in the liquid droplets and the quantity of the protein antigens.

In some embodiments, the liquid droplets prepared by using microfluidic control technology are water-in-oil droplets. The mixed liquid of the test sample and the reaction reagent is the water phase, which is enveloped by a layer of oil phase, thereby forming the water-in-oil droplet. In preparation, the oil phase shears the flow of the mixed liquid containing the test sample and the reaction reagent to obtain the water-in-oil droplets, in which the liquid droplets of the mixed liquid are each surrounded by an oil phase layer. In an optional specific example, the oil phase is made of mineral oil. It should be understood that the material for the oil phase is not limited to the mineral oil and can be other oil materials. It should be understood that the reaction reagent can be in liquid form or in powder form. The reaction reagent in powder form can be added with a corresponding amount of water when using. In some other embodiments, the liquid droplets prepared using microfluidic control technology are aqueous droplets made of the mixed liquid of the test sample and the reaction reagent, only including the water phase. In this embodiment, in preparation, the flow of the mixed liquid is sheared by a gas phase (such as air or inert gas) to form the liquid droplets.

Specifically, the quantity of the antigen-antibody complexes encapsulated within the liquid droplet is controlled by regulating the droplet size. In a specific example, the diameter of a qualified liquid droplet ranges from 10 µm to 15 µm. Furthermore, the diameter of a qualified liquid droplet ranges from 0.1 µm to 1 µm. It should be understood that the size of the qualified liquid droplet is not limited to the above ranges and can be adjusted according to the specific protein antigens and the corresponding antibodies. It is to be noted that the size of the liquid droplet refers to the diameter of the water phase droplet made of the mixed liquid of the test sample and the reaction reagent, excluding the thickness of the surrounding phase outside the mixed liquid.

In some embodiments, after forming the mixture of the test sample and the reaction reagent into the liquid droplets, the method further includes a step of detecting the sizes of the liquid droplets formed from the test sample and the reaction reagent and screening out qualified liquid droplets according to the sizes of the liquid droplets. In an embodiment, the reaction reagent further includes a fluorescent dye distinguishable from the marker. Fluorescent distribution of the fluorescent dye is related to the shape of the liquid droplets, that is, the larger the droplet size, the broader the fluorescence distribution. Thus, the fluorescent distribution of the fluorescent dye can be used to screen out the qualified liquid droplets with suitable size. Moreover, distribution uniformity of the fluorescent dye distinguishable from the marker can be used to determine whether the test sample and the reaction reagent are uniformly mixed. The use of the fluorescent dye distinguishable from the marker prevents the fluorescent dye from interfering with the signal corresponding to the subsequent agglutination reaction, ensuring the ability to detect the signal intensity corresponding to the agglutination reaction. In a specific example, the fluorescent dye is fluorescein. It should be understood that the fluorescent dye is not limited to fluorescein and can be other fluorescent dyes distinguishable from the maker. Correspondingly, the step of measuring the sizes of the liquid droplets formed from the test sample and the reaction reagent and screening out the qualified liquid droplets according to the sizes of the liquid droplets includes: detecting fluorescent distribution of the liquid droplets formed from the test sample and the reaction reagent, and screening out the qualified liquid droplets according to the fluorescent distribution. When a liquid droplet formed from the test sample and the reaction reagent is determined as a qualified liquid droplet, the qualified liquid droplet is used in the following detection. When a liquid droplet formed from the test sample and the reaction reagent is determined as an unqualified liquid droplet, the unqualified liquid droplet is discarded.

It should be understood that, in other embodiments, the method for screening out the qualified liquid droplets is not limited to the above-described method involving adding the fluorescent dye to the water phase of the liquid droplets, and can be other methods.

In step S200, after the agglutination reaction, signal intensities of the liquid droplets formed from the test sample and the reaction reagent are detected and analyzed corresponding to the agglutination reaction, so as to determine the quantity of the protein antigens in the test sample.

In some embodiments, the marker for the antibodies is latex microspheres. Within the microenvironments of the liquid droplets, under the action of the electrolyte, the plurality of antigen-antibody complexes undergo agglutination to form clumps, thereby altering the turbidities of the liquid droplets. Consequently, in the step of detecting the signal intensities of the liquid droplets formed from the test sample and the reaction reagent corresponding to the agglutination reaction after the agglutination reaction, what are detected are the turbidities of the liquid droplets formed from the test sample and the reaction reagent. Specifically, what are detected are the grayscale values of the liquid droplets formed from the test sample and the reaction reagent.

In a specific embodiment, the marker for the antibodies is an aggregation-induced emission (AIE) material. The AIE material is characterized in that the more the aggregation, the brighter the emission. Within the microenvironments of the liquid droplets, the light signals are amplified due to the aggregation, thereby further enhancing the sensitivity and specificity of the protein detection and analysis. Hence, in the step of detecting the signal intensities of the liquid droplets formed from the test sample and the reaction reagent corresponding to the agglutination reaction after the agglutination reaction, what are detected are the luminous intensities of the liquid droplets formed from the test sample and the reaction reagent. In a specific example, the AIE material is a polymer, a hydrogen-containing compound, or a hydrocarbon compound. It should be understood that in other embodiments, the AIE material is not limited to the above examples. Furthermore, the signals corresponding to the agglutination reaction within the liquid droplets formed from the test sample and the reaction reagent are not limited to the turbidities or the luminous intensities, but can be other signals.

By detecting the signal intensities of the liquid droplets formed from the test sample and the reaction reagent corresponding to the agglutination reaction, and counting the number of the liquid droplets, the plurality of signal intensities of the liquid droplets formed from the test sample and the reaction reagent are obtained. By comparing these signal intensities with the signal intensity of a liquid droplet without the antigen-antibody complex, whether the corresponding liquid droplet includes the antigen-antibody complex and/or the quantity of the antigen-antibody complexes included in the liquid droplet can be determined. Specifically, if the signal intensity of the liquid droplet formed from the test sample and the reaction reagent is greater than the signal intensity of the liquid droplet without the antigen-antibody complex, it indicates the presence of the antigen-antibody complex within that liquid droplet. Conversely, if the signal intensity of the liquid droplet formed from the test sample and the reaction reagent is comparable to the signal intensity of the liquid droplet without the antigen-antibody complex, it indicates the absence of the antigen-antibody complex within that liquid droplet. Therefore, in qualitative detection, once there is a liquid droplet, in the liquid droplets formed from the test sample and the reaction reagent, whose signal intensity is greater than that of the liquid droplet without the antigen-antibody complex, it can be determined that the test sample includes the protein antigens.

In an embodiment, the above-described detection method is a qualitative method. Correspondingly, the step of after the agglutination reaction, detecting signal intensities of the liquid droplets formed from the test sample and the reaction reagent corresponding to the agglutination reaction, thereby determining the quantity of the protein antigens in the test sample, includes:
after the agglutination reaction, detecting the signal intensities of the liquid droplets formed from the test sample and the reaction reagent corresponding to the agglutination reaction, thereby obtaining the signal intensities of the liquid droplets formed from the test sample and the reaction reagent; and
determining whether or not any liquid droplet in the liquid droplets formed from the test sample and the reaction reagent includes the antigen-antibody complex according to a difference between the signal intensity of a liquid droplet formed from the test sample and the reaction reagent and the signal intensity of a liquid droplet without the antigen-antibody complex, thereby determining the presence or absence of the protein antigens in the test sample.

The liquid droplet formed from the test sample and the reaction reagent includes the antigen-antibody complex when the signal intensity of the liquid droplet is greater than the signal intensity of the liquid droplet without the antigen-antibody complex. The liquid droplet formed from the test sample and the reaction reagent does not include the antigen-antibody complex when the signal intensity of the liquid droplet is smaller than or equal to the signal intensity of the liquid droplet without the antigen-antibody complex. It should be understood that when mentioning that the signal intensity of the liquid droplet formed from the test sample and the reaction reagent is greater than the signal intensity of the liquid droplet without the antigen-antibody complex, the "greater" refers to greater with a reliability, excluding systematic errors, and is not just in terms of simple numerical comparison. In some embodiments, the above-described detection method is a quantitative method for detecting the protein antigens. In this case, the method further includes detecting the signal intensity of a standard sample corresponding to the agglutination reaction, thereby determining the concentration of the protein antigens in the test sample. It is to be noted that the concentration of the protein antigens in the standard sample is known in advance.

In a specific embodiment, the step of detecting the signal intensity of the standard sample corresponding to the agglutination reaction, thereby determining the concentration of the protein antigens in the test sample, includes:
mixing the standard sample, having a same volume as the test sample, with the reaction reagent, and forming the mixture of the standard sample and the reaction reagent into a plurality of liquid droplets through microfluidic control technology, wherein the volume and type of the reaction reagent mixed with the standard sample are identical to those of the reaction reagent mixed with the test sample;
after the agglutination reaction, detecting and totalizing signal intensities of the liquid droplets formed from the standard sample and the reaction reagent corresponding to the agglutination reaction, thereby obtaining the signal intensity of the standard sample (i.e., the signal intensity corresponding to the agglutination reaction of the standard sample);
according to the signal intensities of the liquid droplets formed from the test sample and the reaction reagent corresponding to the agglutination reaction after the agglutination reaction, totalizing the signal intensities of the liquid droplets formed from the test sample and the reaction reagent, thereby obtaining the signal intensity of the test sample; and
calculating the concentration of the protein antigens in the test sample from the concentration of the protein antigens in the standard sample, the signal intensity of the standard sample, and the signal intensity of the test sample.

Specifically, the concentration of the protein antigens in the test sample equals to the ratio of the signal intensity of the test sample to the signal intensity of the standard sample, multiplied by the concentration of the protein antigens in the standard sample.

It should be understood that in the step of totalizing the signal intensities of the liquid droplets formed from the standard sample and the reaction reagent, only the signal intensities of the liquid droplets including the antigen-antibody complex(es) are taken into account. Correspondingly, in the step of totalizing the signal intensities of the liquid droplets formed from the test sample and the reaction reagent, only the signal intensities of the liquid droplets including the antigen-antibody complex(es) are taken into account.

In another embodiment, the above-described detection method is a quantitative detection method, and the detection method further includes:
mixing the plurality of standard samples, having gradient-varied protein antigen concentrations, respectively with the reaction reagent;
forming the mixtures each into a plurality of liquid droplets through microfluidic control technology;
detecting the signal intensities of the liquid droplets corresponding to the agglutination reaction;
creating a relationship curve between protein antigen concentrations and signal intensities corresponding to the agglutination reaction; and
calculating the concentration of the protein antigens in the test sample from the signal intensity of the test sample corresponding to the agglutination reaction and the relationship curve.

In the above-described method for detecting protein antigens, through microfluidic control technology, a plurality of liquid droplets including the plurality of antigen-antibody complexes and electrolyte are formed. Within the microenvironments of the liquid droplets, the plurality of antigen-antibody complexes undergo agglutination reaction due to the ions dissociated from the electrolyte. The quantity of the protein antigens in the test sample is determined by detecting the signal intensities corresponding to the agglutination reaction. A traditional protein antigen detection method may involve microfluidic control technology and a sandwich assay. For example, the plurality of liquid droplets as reactors are formed on a single chip, and stepwise detections for a sample, captured antibodies, and labeled antibodies are achieved by using liquid droplet fusion technology, followed by bead collection through centrifugation to complete final signal detection. Whereas, such a method cannot achieve digitized detection. Compared to the traditional method, the above-described detection method is simple as it does not require washing and the plurality of rounds of sample additions, making the operation more convenient; moreover, the above-described detection method consumes a decreased amount of reagent with a reduced cost. Additionally, the detection method creates a microenvironment using the liquid droplet, and the liquid droplet has a large specific surface area in microscopic view. The heat and substance transfer in the liquid droplet requires less time, which is conducive to reducing reaction time, thereby expediting the immune reaction. In addition, a minute reaction or change in the liquid droplets can be amplified and easily detected, thereby achieving a high sensitivity. Moreover, the microfluidic control technology can rapidly generate a great number of uniform liquid droplets, enabling numerous parallel reactions to be conducted in a short time with high reproducibility.

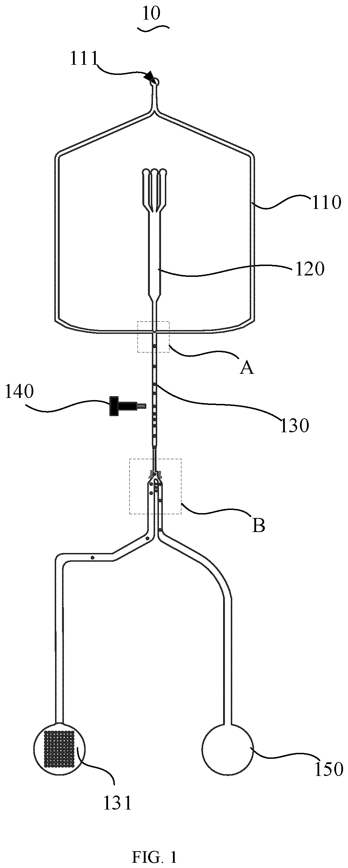

Referring to FIG. 1, the quantity of the protein antigens in the test sample can be detected on the basis of detecting the signals corresponding to the agglutination reaction of the antigen-antibody complexes under the action of ions within the liquid droplets formed by the microfluidic control technology. The present application further provides an embodiment of a protein antigen detection system, including a microfluidic chip 10 for detecting the protein antigens and a detection component. The microfluidic chip 10 includes a droplet-forming component, a droplet channel 130, and a detection chamber 131.

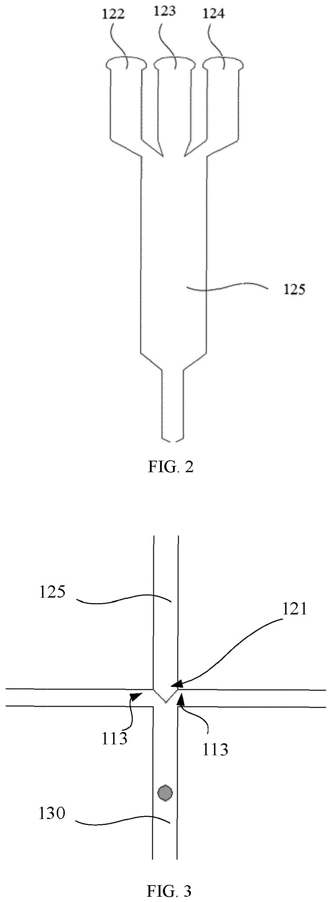

Referring also to FIG. 2 and FIG. 3, the droplet-forming component is configured to form a mixture of a sample and a reaction reagent into a plurality of liquid droplets. The droplet-forming component includes a first-phase channel 110 and a reagent channel 120. The first-phase channel 110 includes a first-phase inlet 111 and two first-phase outlets 113. The two first-phase outlets 113 are spaced from and opposite to each other. The first-phase inlet 111 is in communication with both of the two first-phase outlets 113. The reagent channel 120 includes a reagent inlet and a reagent outlet 121 in communication with the reagent inlet. The reagent outlet 121 is located adjacent to the two first-phase outlets 113, allowing the solution flowing out from the reagent outlet 121 to be enveloped by the first phase flowing out from the first-phase outlets 113, forming a liquid droplet. In a specific embodiment, the material of the first phase can be an oil phase or a gas phase. More specifically, the first-phase channel 110 is a three-way tubular channel, and the first phase flows into one branch and flows out from the other two branches. The first-phase outlets 113 are located at the ends of the branches of the first-phase channel 110 configured for the outflow of the first phase, while the reagent outlet 121 is located in the mid-perpendicular line of the line connected between the two first-phase outlets 113. This arrangement of the reagent outlet 121 and the first-phase outlets 113 facilitates the uniform envelopment of the first phase around the solution flowing out from the reagent outlet 121.

In the embodiment shown in the figure, the first-phase channel 110 extends to form a pentagon, with the two first-phase outlets 113 located in the same edge of the pentagon. The first-phase inlet 111 is located in a line connected between the intersection of the adjacent edges of the pentagon opposite to the edge of the pentagon where the first-phase outlets 113 are located and the reagent outlet 121. The first-phase outlets 113, the reagent outlet 121, and the first-phase inlet 111 are approximately aligned in a straight line. It is to be understood that, in other embodiments, the first-phase channel 110 is not limited to the above-described pentagonal shape but can be of other shapes such as a diamond, a rectangle, etc. The first-phase inlet 111 is not limited to the above-described location. For example, the first-phase inlet 111 can be located at the left or right of the mid-perpendicular of the edge where the first-phase outlets 113 are located. In this case, the spacing between the two first-phase outlets 113 can be located at the right or left, making the two outflow branches roughly equal in length. It should be understood that, the number of the first-phase inlet 111 is not limited to one but can be plural. Similarly, the number of the first-phase outlets 113 is not limited to two but can be other numbers, as long as the required liquid droplets can be formed.

In the embodiment shown in the figure, the reagent channel 120 is a four-way tubular channel, and solutions in the four branches of the four-way tubular channel flow along the same direction. Hence, the solutions introduced from different branches all can exit from the branch for outflow of the solution. In the embodiment shown in the figure, the four branches of the reagent channel 120 are respectively a labeled antibody branch 122 (the left branch in the figure), a test sample branch 123 (the middle branch in the figure), an auxiliary agent branch 124 (the right branch in the figure), and a mixing branch 125 (the branch adjacent to the droplet channel 130). The test sample branch 123 is configured for introducing a test sample, the labeled antibody branch 122 is configured for introducing marker-labeled antibodies, the auxiliary agent branch 124 is configured for introducing an electrolyte providing agent and other reagents, and the mixing branch 125 is configured for mixing the test sample from the test sample branch 123, the marker-labeled antibodies from the labeled antibody branch 122, and the electrolyte providing agent and other reagents from the auxiliary agent branch 124. It should be understood that in other embodiments, the branches of the reagent channel 120 is not limited to the above-described four branches but can be in other quantities. For example, the reagent channel 120 can be a channel without branches. In this case, the test sample can be pre-mixed with the reaction reagent and introduced into the reagent channel 120. Alternatively, the reagent channel 120 can be a three-way tubular channel, and the test sample and the reaction reagent are separately introduced from two of the three branches, and the remaining branch is configured for the outflow of the mixture of the test sample and the reaction reagent.

It should be understood that by increasing the length of the mixing branch 125 configured for mixing the liquids flowing from other branches, the uniformity of the mixing of the reaction reagent with the test sample can be improved. For example, the mixing branch 125 can be in an S-shape or a wave-shape.

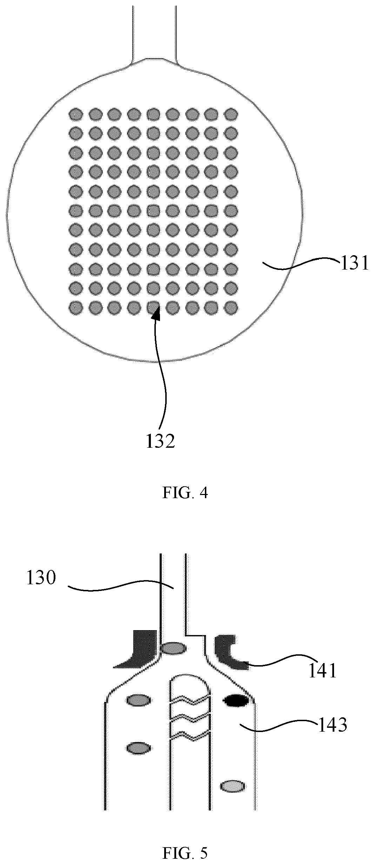

Referring to FIG. 1 and FIG. 4, the droplet channel 130 is configured for receiving the liquid droplets formed by the droplet-forming component. The droplet channel 130 is in communication with the detection chamber 131, allowing the liquid droplets formed by the droplet-forming component to enter the detection chamber 131 through the droplet channel 130. Specifically, the outlet of the droplet channel 130 is in communication with the inlet of the detection chamber 131. In the embodiment shown in FIG. 4, the detection chamber 131 includes a plurality of spaced detection wells 132. The liquid droplets flowing out from the droplet channel 130 are distributed into different detection wells 132. Each detection well 132 corresponds to one liquid droplet, thereby enabling different liquid droplets to occupy different detection wells 132. After the agglutination reaction, the signal intensities of the liquid droplets in different detection wells 132 corresponding to the agglutination reaction are detected, thereby obtaining the signal intensities of the individual liquid droplets corresponding to the agglutination reaction. In a specific example, the detection wells 132 are arranged in an array. In some other embodiments, the detection chamber 131 can include only a single detection well 132, and the distance from the droplet formation position to the detection well 132 is increased to allow the liquid droplet to complete the agglutination reaction when entering the detection well 132. In this case, after detecting the signal corresponding to the agglutination reaction, the liquid droplet can be discarded, and the detection well 132 in the detection chamber 131 can be cleaned.

In some embodiments, the detection chamber 131 is pre-filled with a second phase that is immiscible with water and different from the first phase. For example, when the first phase is a gas phase, the second phase can be an oil phase. In this case, as liquid droplets fall into the detection chamber 131, a more intact droplet shape can be formed, facilitating subsequent detection. For another example, when the first phase is an oil phase, the second phase can be a gas phase.

The detection component is configured for detecting signal intensities of the liquid droplets after the agglutination reaction within the microfluidic chip 10 corresponding to the agglutination reaction. The detection component includes a first detector, which is configured for detecting the signal intensities of the liquid droplets in the detection chamber 131 corresponding to the agglutination reaction.

Referring to FIG. 1 and FIG. 5, in some embodiments, the above-described microfluidic chip 10 further includes a screening channel 143. One end of the screening channel 143 is in communication with the droplet channel 130, allowing the liquid droplets from the droplet channel 130 to flow into the screening channel 143. The other end of the screening channel 143 is divided into two branches, one of which allows qualified liquid droplets to flow, and the other of which allows disqualified liquid droplets to flow.

Correspondingly, the protein antigen detection system as described above further includes a droplet-screening component. The droplet-screening component is configured for screening out qualified liquid droplets from the liquid droplets formed by the droplet-forming component. Specifically, the droplet-screening component includes a second detector 140 and a screening member 141. The second detector 140 is located adjacent to the droplet channel 130 and configured for detecting signals reflecting the size of the liquid droplets formed by the droplet-forming component and forward these signals to the screening member 141. In an example, the second detector 140 is configured for detecting fluorescent signals and feeding these fluorescent signals back to the screening member 141. It is to be noted that the second detector 140 can be replaced corresponding to the signal to be detected. The screening member 141 is located adjacent to the outlet of the droplet channel 130 and is configured for screening out the qualified liquid droplets according to the fluorescent signals fed back by the second detector 140, and directing the qualified liquid droplets to the detection chamber 131 for subsequent detection by the detection component. In some embodiments, the second detector 140 can be omitted. In this case, the screening member 141 can screen the liquid droplets on the basis of their charge (similar to flow cytometry and droplet sorting technique).

In some embodiments, the above-described microfluidic chip 10 further includes a waste liquid reservoir 150, which is configured to collect disqualified liquid droplets directed by the screening member 141.

When using the protein antigen detection system as described above, the test sample and the reaction reagent are introduced into the droplet-forming component of the microfluidic chip 10. The mixture of the test sample and the reaction reagent is successively formed into liquid droplets encapsulating antigen-antibody complexes and a certain concentration of electrolyte under the action of the droplet-forming component. When the liquid droplets generated by the droplet-forming component flow into the liquid droplet channel 130, the detection component detects the signal intensities of the liquid droplets corresponding to the agglutination reaction, totalizes the signal intensities of the liquid droplets corresponding to the agglutination, and determines the quantity of the protein antigens in the test sample according to the result of the totalizing.

More specifically, in the embodiment shown in the figures, the first phase is introduced from the first-phase inlet 111. The test sample is introduced from a test sample inlet of the reagent channel, the marker-labeled antibodies are introduced from a labeled antibody inlet of the reagent channel, and a mixture of the electrolyte providing agent and the fluorescent dye is introduced from an inlet of the reagent channel for introducing the electrolyte providing agent and the fluorescent dye. The test sample, the marker-labeled antibodies, the electrolyte, and the fluorescein are mixed during flowing in a direction towards the droplet channel 130 (vertically downward in the figure). The mixed liquid from the reagent outlet 121 and the first phase from the first-phase outlet 113 cooperatively form the liquid droplets. The formed liquid droplets enter the droplet channel 130. The luminous intensities of the liquid droplets flowing in the droplet channel 130 are detected by the second detector 140. The detected luminous intensities of the liquid droplets are fed back to the screening member 141 by the second detector 140. Based on the received luminous intensity information, the screening member 141 sorts the liquid droplets into the branches for the qualified or disqualified liquid droplets. The liquid droplets flow into the detection wells 132 of the detection chamber 131 through the branch for the qualified liquid droplets. After the agglutination reaction of the liquid droplets in the detection wells 132 of the detection chamber 131, the signal intensities of the liquid droplets in the detection wells 132 corresponding to the agglutination reaction are detected by the first detector. The liquid droplets are successively formed when the mixed liquid continuously flows from the reagent outlet 121 and is continuously enveloped by the first phase. The formed liquid droplets enter the waste liquid reservoir 150 or different detection wells 132 along the above-described paths. Once there are no more liquid droplets entering the detection chamber 131, the signal intensities of the liquid droplets in the detection chamber 131 corresponding to the agglutination reaction is totalized and analyzed, thereby determining the presence or absence of the protein antigens and the quantity of the protein antigens in the test sample. The specific analysis of signal intensity refers to step S200 in the protein antigen detection method based on microfluidic droplet technology.

The microfluidic chip 10 for detecting protein antigens is convenient to use as it does not require multiple rounds of sample additions and washing. Moreover, the microfluidic chip 10 can offer fast reaction speed, high sensitivity, and high reproducibility by adopting microenvironments of the liquid droplets.

Based on the above, an embodiment of the present application provides a protein antigen detection kit. The detection kit includes the microfluidic chip for detecting protein antigens in any of the above-described embodiments and a reaction reagent. The reaction reagent includes marker-labeled antibodies and an electrolyte providing agent. The marker-labeled antibodies can specifically bind to protein antigens in the test sample, forming antigen-antibody complexes. The electrolyte providing agent is configured to induce agglutination reaction of the antigen-antibody complexes.

In some embodiments, the reaction reagent further includes a fluorescent dye distinguishable from the marker on the antibodies.

In some embodiments, the detection kit further includes a standard sample.

In some embodiments, the protein antigen detection kit is a detection kit for detecting C-reactive protein (CRP). In this case, the detection kit includes the microfluidic chip for detecting protein antigens in any of the above-described embodiments and a reaction reagent. The reaction reagent includes C-reactive protein antibodies labeled with latex microspheres or an aggregation-induced emission material, a fluorescent dye distinguishable from the aggregation-induced emission material, and an electrolyte providing agent.

The above-described protein antigen detection kit includes the microfluidic chip for detecting protein antigens and the reaction reagent. Through the cooperation of the microfluidic chip for detecting protein antigens and the reaction reagent, there is no need for multiple rounds of sample additions and washing steps, resulting in complicated operations. Additionally, the protein antigen detection kit adopting the microfluidic chip for detecting protein antigens inherits the advantages of the chip.

The embodiments described above represent just a few implementations of this invention. They are intended to provide specific and detailed understandings of the technical solutions of this invention but do not limit the scope of the patent protection. It should be noted that within the conceptual scope of this invention, various modifications and improvements can be made by those skilled in the art. These modifications and improvements are all within the scope of protection for this invention.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present disclosure.

The above-described embodiments are only several implementations of the present invention, and the descriptions are relatively specific and detailed, but they should not be construed as limiting the scope of the present invention. It should be understood by those of ordinary skill in the art that various modifications and improvements can be made without departing from the concept of the present invention, and all fall within the protection scope of the present invention. Therefore, the patent protection of the present invention shall be defined by the appended claims, and the specification and the drawings can be used to explain the content of the claims.

## Claims

1. A method for detecting protein antigens, comprising following steps:
mixing a test sample with a reaction reagent, and forming a mixture of the test sample and the reaction reagent into a plurality of liquid droplets through microfluidic control technology, wherein the reaction reagent comprises marker-labeled antibodies and an electrolyte providing agent, the marker-labeled antibodies are adapted to specifically bind to the protein antigens in the test sample, forming a plurality of antigen-antibody complexes, the plurality of antigen-antibody complexes are encapsulated within the liquid droplets, and the plurality of antigen-antibody complexes are capable of undergoing an agglutination reaction under an action of the electrolyte providing agent; and
after the agglutination reaction, detecting and analyzing signal intensities of the liquid droplets corresponding to the agglutination reaction, thereby determining the quantity of the protein antigens in the test sample.

2. The method according to claim 1, wherein the marker is latex microspheres, and the step of detecting signal intensities of the liquid droplets corresponding to the agglutination reaction after the agglutination reaction comprises detecting turbidities of the liquid droplets.

3. The method according to claim 1, wherein the marker is an aggregation-induced emission material, and the step of detecting signal intensities of the liquid droplets corresponding to the agglutination reaction after the agglutination reaction comprises detecting luminous intensities of the liquid droplets.

4. The method according to claim 1, after the step of forming the mixture of the sample and the reaction reagent into the plurality of liquid droplets, further comprising a step of detecting sizes of the liquid droplets formed from the test sample and the reaction reagent and screening out qualified liquid droplets according to the sizes of the liquid droplets.

5. The method according to claim 4, wherein the reaction reagent further comprises a fluorescent dye distinguishable from the marker; the step of detecting sizes of the liquid droplets formed from the test sample and the reaction reagent and screening out qualified liquid droplets according to the sizes of the liquid droplets comprises:
detecting fluorescent distribution of the liquid droplets formed from the test sample and the reaction reagent; and
screening out the qualified liquid droplets based on the fluorescent distribution.

6. The method according to any one of claims 1 to 5, being a quantitative detection method for the protein antigens, and further comprising following steps:
mixing a standard sample for the protein antigens, having a same volume as the test sample, with the reaction reagent, and forming the mixture of the standard sample and the reaction reagent into a plurality of liquid droplets through microfluidic control technology;
after the agglutination reaction, detecting and totalizing signal intensities of the liquid droplets formed from the standard sample and the reaction reagent corresponding to the agglutination reaction, thereby obtaining a signal intensity of the standard sample;
after the agglutination reaction, detecting the signal intensities of the liquid droplets formed from the test sample and the reaction reagent corresponding to the agglutination reaction, totalizing the signal intensities of the liquid droplets formed from the test sample and the reaction reagent, thereby obtaining a signal intensity of the test sample; and
calculating a concentration of the protein antigens in the test sample from a concentration of the protein antigens in the standard sample, the signal intensity of the standard sample, and the signal intensity of the test sample.

7. A microfluidic chip for detecting protein antigens, comprising:
a droplet-forming component; and
a droplet channel;
wherein the droplet-forming component is configured to form a mixture of a sample and a reaction reagent into a plurality of liquid droplets, the droplet-forming component comprises a first-phase channel and a reagent channel, the first-phase channel comprises a first-phase inlet and a first-phase outlet, the first-phase inlet is in communication with the first-phase outlet; the reagent channel comprises a reagent inlet and a reagent outlet in communication with the reagent inlet, the reagent outlet is located adjacent to the first-phase outlet, allowing a solution flowing out from the reagent outlet to be enveloped by a first phase flowing out from the first-phase outlet, thereby forming a liquid droplet.

8. The microfluidic chip according to claim 7, further comprising a screening channel, a detection chamber, and a waste liquid reservoir, wherein the screening channel is a three-way channel, one end of the screening channel is in communication with the droplet channel, and the other two ends of the screening channel are in communication with the detection chamber and the waste liquid reservoir respectively.

9. A protein antigen detection kit, comprising the microfluidic chip for detecting protein antigens according to claim 7 or 8 and a reaction reagent, wherein the reaction reagent comprises marker-labeled antibodies and an electrolyte providing agent, the marker-labeled antibodies are adapted to specifically bind to protein antigens in the test sample, forming antigen-antibody complexes, and the electrolyte providing agent is configured to provide conditions for agglutination reaction of the antigen-antibody complexes.

10. A protein antigen detection system, comprising:
the microfluidic chip according to claim 7; and
a detection component, configured to detect signal intensities of the liquid droplets after the agglutination reaction within the microfluidic chip corresponding to the agglutination reaction.
